# EUROPEAN PATENT APPLICATION

(11) **EP 4 442 127 A1**
(43) Date of publication of application: **09.10.2024**
(21) Application number: 22901714.0
(22) Date of filing: 29.11.2022
(51) Int. Cl.: A23L 33/135, C12N 1/20, A23L 27/10, A23L 29/00, A61K 35/74, A61P 37/00, C12R 1/01

(54) **LEUCONOSTOC MESENTEROIDES CJLM119 STRAIN, AND FOOD FERMENTATION COMPOSITION COMPRISING CULTURE PRODUCT THEREOF**

(30) Priority: 30.11.2021 KR 20210168677; 31.10.2022 KR 20220142862
(71) Applicant: CJ CheilJedang Corporation, Seoul 04560 (KR)
(72) Inventor: HONG, Na Youn, Seoul 04560 (KR); JEON, Kyung Chan, Seoul 04560 (KR); JUNG, Hee Kyoung, Seoul 04560 (KR); CHOI, Seung Hye, Seoul 04560 (KR); AHN, Hee Yoon, Seoul 04560 (KR); LEE, Eunyong, Seoul 04560 (KR); ROH, Ji Eun, Seoul 04560 (KR); SON, So-Hyeon, Seoul 04560 (KR)
(74) Representative: Icosa
(86) International application number: PCT/KR2022/019030
(87) International publication number: WO 2023/101356

(57) **Abstract**

The present disclosure relates to a composition for immune enhancement or gut health improvement, which is fermented with a composition for fermentation containing any one or more selected from a *Leuconostoc mesenteroides* CJLM119 strain or a culture thereof and contains any one or more selected from a culture of *Leuconostoc mesenteroides* CJLM119 strain, a lysate thereof, an extract thereof, or a component derived therefrom.

## Description

### [Technical Field]

The present disclosure relates to a composition for immune enhancement or gut health improvement, which is fermented with a composition for fermentation comprising any one or more selected from a *Leuconostoc mesenteroides* CJLM119 strain or a culture thereof and comprises any one or more selected from a culture of *Leuconostoc mesenteroides* CJLM119 strain, a lysate thereof, an extract thereof, or a component derived therefrom.

### [Background Art]

Fermented foods refer to foods or beverages whose ingredients have been converted through the growth of specific microorganisms and the action of enzymes. Beneficial bacteria, including lactic acid bacteria, are reported to increase the bioavailability of nutrients by converting the chemical components that make up food ingredients during the fermentation process, enhance food safety as biological preservatives, decompose toxic substances and nutritional inhibitors, and produce bioactive substances (Tamang JP, et al., Front Microbiol (2016), 7, 578.).

Among the lactic acid bacteria, *Leuconostoc mesenteroides* is known as a representative lactic acid bacterium involved in lactic acid fermentation of vegetables and is found in fermented vegetables such as kimchi.

### [Disclosure]

### [Technical Problem]

The present disclosure provides a composition for immune enhancement or gut health improvement, which is fermented with a composition for fermentation comprising any one or more selected from a *Leuconostoc mesenteroides* CJLM119 strain or a culture thereof and comprises any one or more selected from a culture of *Leuconostoc mesenteroides* CJLM119 strain, a lysate thereof, an extract thereof, or a component derived therefrom.

### [Technical Solution]

An object of the present disclosure is to provide a composition for fermentation, comprising any one or more selected from a *Leuconostoc mesenteroides* CJLM119 strain or a culture thereof.

Another object of the present disclosure is to provide a food composition for immune enhancement, comprising a fermentation product fermented with a *Leuconostoc mesenteroides* CJLM119 strain or a culture thereof.

Still another object of the present disclosure is to provide a food composition for gut health improvement, comprising a fermentation product fermented with a *Leuconostoc mesenteroides* CJLM119 strain or a culture thereof.

Still another object of the present disclosure is to provide a health functional food for immune enhancement, comprising a fermentation product fermented with a *Leuconostoc mesenteroides* CJLM119 strain or a culture thereof.

Still another object of the present disclosure is to provide a health functional food for gut health improvement, comprising a fermentation product fermented with a *Leuconostoc mesenteroides* CJLM119 strain or a culture thereof.

Still another object of the present disclosure is to provide a method for increasing an immune enhancing effect of a composition, the method including fermenting the composition with a *Leuconostoc mesenteroides* CJLM119 strain or a culture thereof.

Still another object of the present disclosure is to provide a method for increasing a gut health improving effect of a composition, the method including fermenting the composition with a *Leuconostoc mesenteroides* CJLM119 strain or a culture thereof.

### [Advantageous Effects]

The composition of the present disclosure has an immune enhancing effect and a gut health improving effect.

### [Brief Description of Drawings]

FIG. 1 illustrates the results of examining cytotoxicity after treating J774A.1 cells, which are mouse immune cells, with fermented compositions of Preparation Examples 1 to 3 and Comparative Examples 1 and 2;
FIG. 2 illustrates the results of examining cytotoxicity after treating J774A.1 cells, which are mouse immune cells, with fermented compositions comprising a *Leuconostoc mesenteroides* CJLM119 strain at 5 × 10⁶ cfu/mL (fermented composition A), 10⁶ cfu/mL (fermented composition B), and 5 × 10⁵ cfu/mL (fermented composition C), and supernatants thereof;
FIG. 3 illustrates the results of examining cytotoxicity after treating J774A.1 cells, which are mouse immune cells, with fermented compositions comprising a *Leuconostoc mesenteroides* CJLM119 or KCTC 3505 strain at 1.25 × 10⁶ cfu/mL and 6.25 × 10⁶ cfu/mL;
FIG. 4 illustrates the results of examining cytotoxicity after treating J774A.1 cells, which are mouse immune cells, with fermented compositions comprising a *Leuconostoc mesenteroides* CJLM119 or KCTC 3505 strain at 1.25 × 10⁶ cfu/mL, 2.5 × 10⁶ cfu/mL, and 5.0 × 10⁶ cfu/mL;
FIG. 5 illustrates the results of analyzing the expression level of TNF-α (tumor necrosis factor-α) in J774A.1 cells, which are mouse immune cells, treated with fermented compositions of Preparation Examples 1 to 3 and Comparative Examples 1 and 2 (***p < 0.001);
FIG. 6 illustrates the results of analyzing the expression level of IL-6 (interleukin-6) in J774A.1 cells, which are mouse immune cells, treated with fermented compositions of Preparation Examples 1 to 3 and Comparative Examples 1 and 2 (***p < 0.001);
FIG. 7 illustrates the results of analyzing the expression level of TNF-α after treating J774A.1 cells, which are mouse immune cells, with fermented compositions comprising a *Leuconostoc mesenteroides* CJLM119 strain at 5 × 10⁶ cfu/mL, 10⁶ cfu/mL, and 5 × 10⁵ cfu/mL (***p < 0.001, **p < 0.01);
FIG. 8 illustrates the results of analyzing the expression level of IL-6 after treating J774A.1 cells, which are mouse immune cells, with fermented compositions comprising a *Leuconostoc mesenteroides* CJLM119 strain at 5 × 10⁶ cfu/mL, 10⁶ cfu/mL, and 5 × 10⁵ cfu/mL (***p < 0.001, **p < 0.01);
FIG. 9 illustrates the results of analyzing the expression level of TNF-α after treating J774A.1 cells, which are mouse immune cells, with fermented compositions comprising a *Leuconostoc mesenteroides* CJLM119 or KCTC 3505 strain at 1.25 × 10⁶ cfu/mL and 6.25 × 10⁶ cfu/mL (***p < 0.001, ##p < 0.01, #p < 0.05);
FIG. 10 illustrates the results of analyzing the expression level of TNF-α after treating J774A.1 cells, which are mouse immune cells, with fermented compositions comprising a *Leuconostoc mesenteroides* CJLM119 or KCTC 3505 strain at 1.25 × 10⁶ cfu/mL, 2.5 × 10⁶ cfu/mL, and 5.0 × 10⁶ cfu/mL (**p < 0.01, *p < 0.05);
FIG. 11 illustrates the results of analyzing the expression level of IL-6 after treating J774A.1 cells, which are mouse immune cells, with fermented compositions comprising a *Leuconostoc mesenteroides* CJLM119 or KCTC 3505 strain at 1.25 × 10⁶ cfu/mL and 6.25 × 10⁶ cfu/mL (***p < 0.001, ##p < 0.01, #p < 0.05);
FIG. 12 illustrates the results of analyzing the expression level of IL-6 after treating J774A.1 cells, which are mouse immune cells, with fermented compositions comprising a *Leuconostoc mesenteroides* CJLM119 or KCTC 3505 strain at 1.25 × 10⁶ cfu/mL, 2.5 × 10⁶ cfu/mL, and 5.0 × 10⁶ cfu/mL (***p < 0.001, **p < 0.01, *p < 0.05); and
FIG. 13 illustrates the results of comparing the effect of inhibiting the growth of harmful bacteria of the fermented composition of Preparation Example 4. Terms: fermented composition, Fermentation; supernatant of fermented composition, Metabolite.

### [Best Modes for Carrying out the Invention]

The present disclosure is explained in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description below. Additionally, a number of papers and patent documents are referenced, and citations are indicated throughout this specification. The disclosures of the cited papers and patent documents are incorporated by reference into this specification in their entirety to more clearly explain the content of the present disclosure and the level of technical field to which the present disclosure belongs.

An aspect of the present disclosure provides a composition for fermentation, comprising any one or more selected from a *Leuconostoc mesenteroides* CJLM119 strain or a culture thereof.

The strain of the present disclosure may be a strain of *Leuconostoc sp.,* specifically *Leuconostoc mesenteroides,* more specifically a *Leuconostoc mesenteroides* CJLM119 strain (US 2019-0297908 A1) deposited with accession number KCTC 13043BP.

In the present disclosure, the term "culture" may refer to the entire medium, which comprises the strain of the present disclosure, its metabolites, extra nutrients and the like, and is obtained by culturing the strain for a certain period of time in a medium capable of supplying nutrients so that the strain can grow and survive in a test tube. The "culture" is a composition comprising a strain, and in the present invention, the culture may be used for the same purpose as the strain. In the present disclosure, the term "culture" may be used interchangeably with "culture solution," "culture supernatant," "conditioned culture solution," or "conditioned medium".

In the present disclosure, the term "culturing" means growing the microorganism under appropriately controlled environmental conditions. The culturing process of the present disclosure may be carried out according to appropriate media and culture conditions known in the art. This culturing process may be easily adjusted and used by those skilled in the art depending on the strain selected. Specifically, the culturing may be batch, continuous, or fed-batch processes, but is not limited thereto.

The carbon source contained in the medium may include sugars and carbohydrates such as glucose, sucrose, lactose, fructose, maltose, starch, and cellulose, oils and fats such as soybean oil, sunflower oil, castor oil, and coconut oil, fatty acids such as palmitic acid, stearic acid, and linoleic acid, alcohols such as glycerol and ethanol, and organic acids such as acetic acid, and these substances may be used individually or in a mixture, but the carbon source is not limited thereto.

The nitrogen source contained in the medium may include organic nitrogen sources such as peptone, yeast extract, broth, malt extract, corn steep liquor, and soybean meal and inorganic nitrogen sources such as urea, ammonium sulfate, ammonium chloride, ammonium phosphate, ammonium carbonate, and ammonium nitrate, and these nitrogen sources may be used singly or in combination, but the nitrogen source is not limited thereto.

The phosphorus source contained in the medium may include monopotassium phosphate, dipotassium phosphate, monosodium phosphate, and disodium phosphate, but is not limited thereto.

The medium may contain metal salts such as magnesium sulfate or iron sulfate as well as amino acids, vitamins, and appropriate precursors. These media or precursors may be added to the culture in a batch or continuous manner, but are not limited thereto.

During culturing, chemicals such as ammonium hydroxide, potassium hydroxide, ammonia, phosphoric acid, and sulfuric acid may be added to the culture in an appropriate manner to adjust the pH of the culture. During culturing, antifoaming agents such as fatty acid polyglycol ester may be used to suppress foam generation. Oxygen or oxygen-containing gas may be injected into the culture in order to maintain the aerobic state of the culture, or gas may not be injected or nitrogen, hydrogen, or carbon dioxide gas may be injected in order to maintain anaerobic and microaerobic states.

The culture may be obtained by culturing the strain of the present disclosure at 10°C to 40°C for 6 to 48 hours, for example, at 20°C to 40°C for 12 to 36 hours, or at 30°C to 40°C for 16 to 24 hours.

The culture of the present disclosure may include a live culture (that is, a resultant product obtained by culturing live bacteria in a medium), a culture obtained by killing live bacteria in the live culture, culture filtrate (that is, a resultant product obtained by removing dead bacteria or live bacteria from the culture), and a concentrate or freeze-dried product thereof.

The culture filtrate may be obtained by leaving the culture solution still for a certain period of time and taking only the upper layer liquid excluding the portion that has settled in the lower layer, by removing bacteria through filtration, or by centrifuging the culture solution to remove sediment at the bottom and taking only the upper liquid. In the present disclosure, the "culture filtrate" may be used interchangeably with "supernatant".

The bacteria may be obtained by centrifuging the culture solution and taking the portion that has settled in the lower layer, or by leaving the culture solution still for a certain period of time and then removing the upper liquid since the bacteria sink to the lower layer of the culture solution by gravity.

The concentrate may be obtained by concentrating the strain culture solution itself or the supernatant obtained through centrifugation or filtration using a filter of the culture solution.

The freeze-dried product may be obtained by freeze-drying the strain culture solution itself or the culture solution.

The composition for fermentation of the present disclosure may be used in the production of various fermented foods, and may be specifically for fermenting condiments such as garlic, red pepper powder, and seasoning, but is not limited thereto.

In a case where fermentation is performed using the composition for fermentation of the present disclosure, a food having an immune enhancing effect and/or a gut health improving effect may be produced, but is not limited thereto.

Another aspect of the present disclosure provides a food composition for immune enhancement, comprising a fermentation product fermented with a *Leuconostoc mesenteroides* CJLM119 strain or a culture thereof.

Still another aspect of the present disclosure provides a food composition for gut health improvement, comprising a fermentation product fermented with a *Leuconostoc mesenteroides* CJLM119 strain or a culture thereof.

The *Leuconostoc mesenteroides* CJLM119 strain and culture are as described above.

The food composition of the present disclosure may include a fermentation product fermented with the composition for fermentation of the previous aspect. As an example, the fermentation product may include a fermentation product fermented with a *Leuconostoc mesenteroides* CJLM119 strain or a culture thereof. Alternatively, the fermentation product may include a fermentation product in which the fermentation product fermented with a *Leuconostoc mesenteroides* CJLM119 strain or a culture thereof is further fermented.

In an exemplary embodiment of the present disclosure, the food composition of the present disclosure may comprise garlic, red pepper powder, a nitrogen source, and the like fermented with the composition for fermentation of the previous aspect.

As used in the present disclosure, the term "fermentation" refers to any activation or process involving enzymatic or metabolic decomposition of organic substances using microorganisms.

In the present disclosure, the term "fermentation product" refers to a resultant product of enzymatic or metabolic decomposition accompanying fermentation using microorganisms. As an example, the fermentation product may be in liquid form. Additionally, the fermentation product may be a crude solution that is a resultant product of fermentation or a supernatant thereof.

In another exemplary embodiment of the present disclosure, the fermentation product of the present disclosure may comprise live bacteria.

In still another exemplary embodiment of the present disclosure, the fermentation product of the present disclosure may undergo additional fermentation. As an example, the fermentation product of the present disclosure may be added to vegetables and the like and undergo additional fermentation, or may be added to a liquid composition and the like and undergo additional fermentation. Examples of the vegetables include cabbage and radish, but are not limited thereto.

The fermentation product of the present disclosure may be a product fermented at 25°C to 40°C using any one or more selected from the strain of the present disclosure or a culture thereof. The fermentation product of the present disclosure may be a product fermented for 10 to 30 hours using any one or more selected from the strain of the present disclosure or a culture thereof.

As an example, the temperature at the time of fermentation of the present disclosure may be in a range consisting of one lower limit selected from 25°C or more, 27°C or more, 28°C or more and 29°C or more and/or one upper limit selected from 40°C or less, 35°C or less, 33°C or less and 31°C or less. The time at the time of fermentation of the present disclosure may be in a range consisting of one lower limit selected from 10 hours or more, 12 hours or more, 14 hours or more, 16 hours or more, and 18 hours or more and/or one upper limit selected from 30 hours or less, 28 hours or less, 27 hours or less, 26 hours or less, and 25 hours or less.

The fermentation product of the present disclosure may be a product in which a composition comprising any one or more selected from the group consisting of garlic, red pepper powder, and a nitrogen source is fermented. As an exemplary embodiment, the composition to be fermented of the present disclosure may be a composition comprising any one or more selected from the group consisting of garlic, red pepper powder, and a nitrogen source, but is not limited thereto. As another exemplary embodiment, the fermentation product of the present disclosure may be obtained by adding the strain of the present disclosure and/or a culture thereof to a composition comprising any one or more selected from the group consisting of garlic, red pepper powder, and a nitrogen source and fermenting the composition.

The fermentation product of the present disclosure may be obtained by adding the strain of the present disclosure and/or a culture thereof at 0.5% to 4% by weight with respect to the total weight of a composition comprising any one or more selected from the group consisting of garlic, red pepper powder, and a nitrogen source.

As an example, the strain of the present disclosure and/or a culture thereof may be contained in a range consisting of one lower limit selected from 1% by weight or more, 1.2% by weight or more, 1.6% by weight or more and 1.8% by weight or more and/or one upper limit selected from 3.5% by weight or less, 2.8% by weight or less, 2.6% by weight or less and 2.2% by weight or less with respect to the total weight of the composition.

The fermentation product of the present disclosure may be a product in which a composition comprising garlic at 4% to 10% by weight with respect to the total weight of the composition is fermented. As an exemplary embodiment, the composition to be fermented of the present disclosure may be a composition comprising any one or more selected from the group consisting of garlic, red pepper powder, and a nitrogen source, but is not limited thereto. As another exemplary embodiment, the composition to be fermented of the present disclosure, which comprises any one or more selected from the group consisting of garlic, red pepper powder, and a nitrogen source, may comprise garlic at 4% to 10% by weight with respect to the total weight of the composition.

As an example, the garlic may be contained in a range consisting of one lower limit selected from 4% by weight or more, 5% by weight or more, 6% by weight or more and 6.5% by weight or more and/or one upper limit selected from 10% by weight or less, 9% by weight or less, 8% by weight or less and 7.5% by weight or less with respect to the total weight of the composition.

The garlic may be contained in the composition of the present disclosure in the form of minced garlic, extract, concentrate, powder or the like, but is not limited thereto, and commercially available garlic and products processed in the form of minced garlic, extract, concentrate, powder or the like may be used without limitation.

As still another exemplary embodiment, the composition to be fermented of the present disclosure, which comprises any one or more selected from the group consisting of garlic, red pepper powder, and a nitrogen source, may comprise red pepper powder at 0.5% to 4% by weight with respect to the total weight of the composition.

As an example, the red pepper powder may be contained in a range consisting of one lower limit selected from 0.5% by weight or more, 1% by weight or more, 1.2% by weight or more, 1.6% by weight or more and 1.8% by weight or more and/or one upper limit selected from 4% by weight or less, 3.5% by weight or less, 2.8% by weight or less, 2.6% by weight or less and 2.2% by weight or less with respect to the total weight of the composition.

As the red pepper powder, peppers may be dried and then ground, or freeze-dried and powdered, but the red pepper powder is not limited thereto, and commercially available red pepper powder may be used without limitation.

As still another exemplary embodiment, the composition to be fermented of the present disclosure, which comprises any one or more selected from the group consisting of garlic, red pepper powder, and a nitrogen source, may comprise a nitrogen source at 0.1% to 1.5% by weight with respect to the total weight of the composition.

As an example, the nitrogen source may be contained in a range consisting of one lower limit selected from 0.3% by weight or more, 0.6% by weight or more, 0.9% by weight or more and 1.1% by weight or more and/or one upper limit selected from 1.45% by weight or less, 1.4% by weight or less, 1.35% by weight or less and 1.3% by weight or less with respect to the total weight of the composition.

The nitrogen source may be edible. The nitrogen source may be a seasoning, for example, TasteNrich^{®} (CJ CheilJedang), but is not limited thereto.

Specifically, the composition of the present disclosure may comprise garlic.

The fermentation product of the present disclosure may be a product in which a composition further comprising any one or more selected from a carbon source or salt is fermented.

In an exemplary embodiment, the composition to be fermented of the present disclosure may comprise a carbon source at 2% to 4% by weight with respect to the total weight of the composition.

As an example, the carbon source may be contained in a range consisting of one lower limit selected from 2.2% by weight or more, 2.4% by weight or more, 2.6% by weight or more and 2.8% by weight or more and/or one upper limit selected from 3.8% by weight or less, 3.6% by weight or less, 3.4% by weight and less or 3.2% by weight or less with respect to the total weight of the composition.

The carbon source may be edible. The carbon source may be fructose, sucrose, glucose, and the like, for example, fructose, specifically crystalline fructose, but is not limited thereto.

As an exemplary embodiment, the composition to be fermented of the present disclosure may comprise salt at 0.1% to 2% by weight with respect to the total weight of the composition.

As an example, the salt may be contained in a range consisting of one lower limit selected from 0.5% by weight or more, 0.8% by weight or more, 1% by weight or more and 1.2% by weight or more and/or one upper limit selected from 1.9% by weight or less, 1.8% by weight or less, 1.7% by weight or less and 1.6% by weight or less with respect to the total weight of the composition.

The fermentation product of the present disclosure may be a product in which a composition further comprising ginger is fermented.

As an exemplary embodiment, the composition to be fermented of the present disclosure may comprise ginger at 0.1% to 1.5% by weight with respect to the total weight of the composition.

As an example, the ginger may be contained in a range consisting of one lower limit selected from 0.3% by weight or more, 0.6% by weight or more, 0.9% by weight or more and 1.1% by weight or more and/or one upper limit selected from 1.45% by weight or less, 1.4% by weight or less, 1.35% by weight or less and 1.3% by weight or less with respect to the total weight of the composition.

The fermentation product of the present disclosure may be a product in which a composition that further comprising water is fermented.

In an exemplary embodiment of the present disclosure, the water may be added until the total weight of the composition becomes 100% by weight after all the raw materials, which are contained in the composition to be fermented of the present disclosure, have been mixed together.

The fermentation product of the present disclosure may comprise any one or more selected from a culture of *Leuconostoc mesenteroides* CJLM119 strain, a lysate thereof, an extract thereof, or a component derived therefrom.

In the present disclosure, the term "lysate" refers to a product obtained by breaking the cell walls of microorganisms contained in the culture solution or a concentrate thereof by chemical or physical force. In the present disclosure, the term "lysate" may be used interchangeably with "disruption product".

In the present disclosure, the term "extract" refers to a material obtained by removing microorganisms from the culture solution or a concentrate thereof.

The component derived from the strain of the present disclosure may include, for example, metabolites derived from the strain, and may include cytoplasmic fractions obtained by disrupting bacteria containing metabolites derived from the strain and the like. In addition, the form of strain suitable to be contained in the food composition, the formulation method, and the method for isolating strain-derived components are well known to those skilled in the art.

In an exemplary embodiment of the present disclosure as described above, the fermentation product of the present disclosure may be a product in which a composition comprising ingredients contained in kimchi sauce (kimchi condiment) is fermented.

The food composition of the present disclosure may have an immune enhancing effect.

In the present disclosure, the term "immune enhancement" refers to the effect of increasing the immune response in various specific or non-specific ways, and may further include an immune regulating effect. The immune enhancing effect may include, for example, protection against external pathogens by increasing the activity of macrophages, removal of dead cells, and an effect of increasing the secretion of immune mediators, and may include the effect of increasing the activity of the immune system by increasing the activity of immune regulatory factors, inhibiting the formation of new blood vessels, and preventing immune-related diseases by increasing the pathogen growth inhibitory power.

Specifically, the immune enhancing effect may be achieved by the effect of increasing the secretion of immune mediators.

For example, the immune mediator may be Th1 type pro-inflammatory cytokines, for example, tumor necrosis factor-α (TNF-α), interleukin-6 (IL-6), and IL-1β, but is not limited thereto.

In an exemplary embodiment of the present disclosure, the composition of the present disclosure may increase the expression of TNF-α or IL-6.

The food composition of the present disclosure may have a gut health improving effect.

In the present disclosure, the term "gut health improvement" refers to the effect of maintaining the intestinal environment in a normal state by controlling the flora of beneficial and harmful bacteria in the intestines. The gut health improving effect may include, for example, the effect of promoting the flora of beneficial bacteria and suppressing the flora of harmful bacteria in the intestines, may include the effect of improving and activating the overall function of the intestines by strengthening the tight junction barrier function of the intestines, may include the effect of increasing the content of short-chain fatty acids in the intestines, and may include the effect of inducing proliferation of intestinal epithelial cells, and the like. In the present disclosure, the "gut health improving effect" may be used interchangeably with the "intestinal regulation effect".

Specifically, the gut health improving effect may be achieved by increasing the flora of beneficial bacteria and suppressing the flora of harmful bacteria in the intestines through the effect of inhibiting the growth of harmful bacteria.

The food composition of the present disclosure may have the effect of inhibiting the growth of harmful bacteria.

The harmful bacteria may be pathogenic microorganisms.

The pathogenic microorganisms are not particularly limited as long as they cause specific diseases upon infection, may be, for example, *Escherichia sp., Staphylococcus sp., Salmonella sp., Shigella sp., Klebsiella sp., Enterobacteriaceae sp., Pseudomonas sp., Moraxella sp., Helicobacter sp., Stenotrophomonas sp., Listeria sp., and Vibrio sp.,* may be, for example, *Escherichia coli, Staphylococcus aureus, Salmonella enteritidis, Salmonella typhi, Shigella dysenteriae, Klebsiella pneumoniae, Pseudomonas aeruginosa, Moraxella catarrhalis, Helicobacter pylori, Stenotrophomonas maltophilia, Listeria monocytogene, Vibrio cholerae* and *Vibrio vulnificus,* and may specifically be *Escherichia coli* or *Staphylococcus aureus,* but are not limited thereto.

The food composition of the present disclosure may comprise a fermentation product fermented with the composition for fermentation of the previous aspect. As an example, the fermentation product may include a fermentation product fermented with a *Leuconostoc mesenteroides* CJLM119 strain or a culture thereof. Alternatively, the fermentation product may include a fermentation product in which the fermentation product fermented with a *Leuconostoc mesenteroides* CJLM119 strain or a culture thereof is further fermented. This is as described above.

As an example, the fermentation product may be in liquid form. Additionally, the fermentation product may be a crude solution that is a resultant product of fermentation or a supernatant thereof.

The fermentation product may further undergo additional fermentation. As an example, the fermentation product of the present disclosure may be added to vegetables and the like and undergo additional fermentation, or may be added to a liquid composition and the like and undergo additional fermentation. Examples of the vegetables include cabbage and radish, but are not limited thereto.

In a case where the fermentation product is a liquid fermentation product, the fermentation product of the present invention may include both the liquid fermentation product and/or a fermentation product in which the liquid fermentation product is further fermented. At this time, the fermentation product obtained through further fermentation may be a liquid or solid fermentation product, but is not limited thereto.

The food composition of the present disclosure may be in the form of a food additive in addition to the food composition, but is not limited thereto.

The food composition of the present disclosure may further comprise food additives, and unless otherwise specified, suitability as a "food additive" can be determined in accordance with the specifications and standards for the relevant item in conformity with the general provisions and general test methods of the food additives code approved by the Food and Drug Safety Administration.

Examples of the items listed in the "food additives code" include chemical compounds such as ketones, glycine, potassium citrate, nicotinic acid, and cinnamic acid, natural additives such as persimmon color, licorice extract, crystalline cellulose, and guar gum, and mixed preparations such as L-glutamate sodium preparations, noodle additive alkaline preparations, preservative preparations, and tar color preparations.

Examples of the food of the present disclosure include beverages such as fruit drinks, vegetable drinks, carbonated drinks, soy milk, yogurt and other lactic acid bacteria drinks, and mixed drinks; kimchi, kimchi sauce (kimchi condiment), fermented foods; confectionery such as bread, rice cake, dried fruit, candy, chocolate, chewing gum, and jam, and ice cream products such as ice cream, frozen desserts, and ice cream powder; dairy products such as milk, low-fat milk, lactose-degraded milk, processed milk, goat milk, fermented milk, butter milk, concentrated milk, milk cream, butter oil, natural cheese, processed cheese, powdered milk, and whey; meat products such as processed meat products, processed egg products, and hamburgers; fish products such as processed fish products such as fish cakes, ham, sausages, and bacon; noodles such as ramen, dried noodles, fresh noodles, fried noodles, luxury dried noodles, improved cooked noodles, frozen noodles, and pasta; seasonings such as soy sauce, soybean paste, red pepper paste, chunjang, cheonggukjang, mixed paste, vinegar, sauces, tomato ketchup, curry, and dressing, but are not limited thereto.

In addition to the above, the food composition of the present disclosure may comprise various nutrients, vitamins, electrolytes, flavors, colorants, pectic acid and its salts, alginic acid and its salts, organic acids, protective colloidal thickeners, pH adjusters, stabilizers, preservatives, glycerin, alcohols, carbonating agents used in carbonated beverages, and the like. Other than these, the food composition of the present disclosure may contain natural fruit juice and pulp for the production of fruit juice drinks and vegetable drinks. These ingredients may be used independently or in combination.

Still another aspect of the present disclosure provides a health functional food for immune enhancement, comprising a fermentation product fermented with a *Leuconostoc mesenteroides* CJLM119 strain or a culture thereof.

Still another aspect of the present disclosure provides a health functional food for gut health improvement, comprising a fermentation product fermented with a *Leuconostoc mesenteroides* CJLM119 strain or a culture thereof.

The *Leuconostoc mesenteroides* CJLM119 strain and culture are as described above.

The fermentation product of the present disclosure may be a product fermented at 32°C to 42°C for 10 to 30 hours using any one or more selected from the strain of the present disclosure or a culture thereof, and this is as described above.

The fermentation product of the present disclosure may be a product in which a composition comprising any one or more selected from the group consisting of garlic, red pepper powder, and a nitrogen source is fermented, and this is as described above.

The fermentation product may be a product in which a composition comprising garlic at 5% to 9% by weight with respect to the total weight of the composition is fermented, and this is as described above.

The fermentation product of the present disclosure may be a product in which a composition that further comprising any one or more selected from a carbon source or salt is fermented, and this is as described above.

The fermentation product of the present disclosure may comprise any one or more selected from a culture of *Leuconostoc mesenteroides* CJLM119 strain, a lysate thereof, an extract thereof, or a component derived therefrom, and this is as described above.

In the present disclosure, the term "health functional food" refers to food that is manufactured and processed using raw materials or ingredients with functional properties useful to the human body in conformity with the Health Functional Food Act (Article 3, Paragraph 1), and "functionality" means adjusting nutrients to the structure and function of the human body or obtaining useful effects for health purposes such as physiological effects (Article 3, Paragraph 2).

The health functional food of the present disclosure may be in the form of a food additive.

In a case of using the health functional food of the present disclosure as a food additive, the health functional food may be added as a composition as it is or used together with other foods or food ingredients, and may be appropriately used according to conventional methods. The active ingredient may be used appropriately depending on its purpose of use (prevention or improvement). Generally, when a food or beverage is manufactured, the health functional food composition of the present invention is added in an amount of 15 parts by weight or less, preferably 10 parts by weight or less with respect to the amount of raw materials. However, in the case of long-term intake for health purposes, the amount may be equal to or less than the above range, and the active ingredient may be used in an amount equal to or more than the above range since there is no problem in terms of safety.

The health functional food may further comprise food additives, and this is as described above.

Still another aspect of the present disclosure provides a feed composition for immunity enhancement, comprising a fermentation product fermented with a *Leuconostoc mesenteroides* CJLM119 strain or a culture thereof.

Still another aspect of the present disclosure provides a feed composition for gut health improvement, comprising a fermentation product fermented with a *Leuconostoc mesenteroides* CJLM119 strain or a culture thereof.

The *Leuconostoc mesenteroides* CJLM119 strain and culture are as described above.

The fermentation product of the present disclosure may be a product fermented at 32°C to 42°C for 10 to 30 hours using any one or more selected from the strain of the present disclosure or a culture thereof, and this is as described above.

The fermentation product of the present disclosure may be a product in which a composition comprising any one or more selected from the group consisting of garlic, red pepper powder, and a nitrogen source is fermented, and this is as described above.

The fermentation product may be a product in which a composition comprising garlic at 5% to 9% by weight with respect to the total weight of the composition is fermented, and this is as described above.

The fermentation product of the present disclosure may be a product in which a composition that further comprising any one or more selected from a carbon source or salt is fermented, and this is as described above.

The fermentation product of the present disclosure may comprise any one or more selected from a culture of *Leuconostoc mesenteroides* CJLM119 strain, a lysate thereof, an extract thereof, or a component derived therefrom, and this is as described above.

The feed composition of the present disclosure may be in the form of a feed additive composition in addition to the feed composition.

In a case where the composition of the present disclosure is prepared as a feed additive, the composition may be a 20% to 90% highly concentrated liquid or may be prepared in the form of powder or granules. The feed additive may further contain any one or more among organic acids such as citric acid, fumaric acid, adipic acid, lactic acid, and malic acid; phosphates such as sodium phosphate, potassium phosphate, acid pyrophosphate, and polyphosphate (polymerized phosphate), and natural antioxidants such as polyphenols, catechin, alpha-tocopherol, rosemary extract, vitamin C, green tea extract, licorice extract, chitosan, tannic acid, and phytic acid. In a case of being prepared as feed, the composition may be formulated in the form of normal feed and may contain common feed ingredients together.

The feed and feed additive may further contain grains such as ground or broken wheat, oats, barley, corn and rice; vegetable protein feed, for example, feed containing rape, soybean, and sunflower; animal protein feed, for example, blood meal, meat meal, bone meal and fish meal; and dry ingredients such as sugar and dairy products, for example, various powdered milk and whey powder, and may further contain nutritional supplements, digestion and absorption enhancers, growth promoters, and the like in addition to these.

The feed additive may be administered to animals singly or by being combined with other feed additives in an edible carrier. The feed additives may be easily administered to animals as top dressing, by mixing this directly into animal feed, or in an oral dosage form separate from the feed. In a case where the feed additive is administered separately from animal feed, the feed additive may be combined with a pharmaceutically acceptable edible carrier to prepare an immediate-release or sustained-release formulation as is well known in the art. Such an edible carrier may be solid or liquid, for example, corn starch, lactose, sucrose, soy flakes, peanut oil, olive oil, sesame oil and propylene glycol. In a case where a solid carrier is used, the feed additive may be a tablet, capsule, powder, troche or sugar-containing tablet or a top dressing in microdisperse form. In a case where a liquid carrier is used, the feed additive may be gelatin soft capsules, or a formulation of a syrup, suspension, emulsion, or solution.

The feed and feed additive may comprise auxiliaries, for example, preservatives, stabilizers, wetting agents or emulsifying agents, and solution accelerators. The feed additive may be added to animal feed by injection, spraying, or mixing and used.

The feed or feed additive of the present disclosure may be applied to a number of diets for animals including mammals, poultry and fish.

The feed or feed additive of the present disclosure may be used for mammals such as pigs, cows, horses, sheep, rabbits, goats, rodents, and laboratory rodents such as rats, hamsters, and guinea pigs as well as companion animals (for example, dogs and cats), for poultry such as chickens, turkeys, ducks, geese, pheasants, and quails, and for fish such as carps, crucian carps, and trout, but is not limited thereto.

Still another aspect of the present disclosure provides a method for preparing a composition for immune enhancement, the method including fermenting a composition comprising any one or more selected from the group consisting of garlic, red pepper powder, and a nitrogen source with a *Leuconostoc mesenteroides* CJLM119 strain or a culture thereof.

Still another aspect of the present disclosure provides a method for preparing a composition for gut health improvement, the method including fermenting a composition comprising any one or more selected from the group consisting of garlic, red pepper powder, and a nitrogen source with a *Leuconostoc mesenteroides* CJLM119 strain or a culture thereof.

The *Leuconostoc mesenteroides* CJLM119 strain and culture are as described above.

The composition prepared according to the method of the present disclosure may be a fermentation product.

The fermentation product of the present disclosure may be a product fermented at 32°C to 42°C for 10 to 30 hours using any one or more selected from the strain of the present disclosure or a culture thereof, and this is as described above.

The fermentation product may be a product in which a composition comprising garlic at 5% to 9% by weight with respect to the total weight of the composition is fermented, and this is as described above.

The fermentation product of the present disclosure may be a product in which a composition further comprising any one or more selected from a carbon source or salt is fermented, and this is as described above.

The fermentation product of the present disclosure may comprise any one or more selected from a culture of *Leuconostoc mesenteroides* CJLM119 strain, a lysate thereof, an extract thereof, or a component derived therefrom, and this is as described above.

Still another aspect of the present disclosure provides a method for increasing an immune enhancing effect of a composition, the method including fermenting the composition with a *Leuconostoc mesenteroides* CJLM119 strain or a culture thereof.

Still another aspect of the present disclosure provides a method for increasing a gut health improving effect of a composition, the method including fermenting the composition with a *Leuconostoc mesenteroides* CJLM119 strain or a culture thereof.

The *Leuconostoc mesenteroides* CJLM119 strain, culture, immune enhancement, and gut health improvement are as described in the previous aspects.

### [Modes for Carrying out the Invention]

Hereinafter, the present disclosure will be described in more detail with reference to Examples. However, the following Examples are merely preferred embodiments for illustrating the present disclosure and are therefore not intended to limit the scope of the present disclosure thereto. Meanwhile, technical matters not described in this specification can be fully understood and easily implemented by those skilled in the technical field of the present disclosure or a similar technical field. Additionally, a number of papers and patent documents are referenced and citations are indicated throughout this specification. The disclosures of the cited papers and patent documents are incorporated by reference into this specification in their entirety to more clearly explain the content of the present disclosure and the level of technical field to which the present disclosure belongs.

### Preparation Example: Preparation of fermented composition

First, compositions were each prepared by mixing raw materials excluding the strain according to Table 1 below. When the compositions were prepared, garlic was added at 7% by weight, ginger at 1.2% by weight, red pepper powder at 2% by weight, a carbon source (crystalline fructose) at 3% by weight, salt at 1.5% by weight, and seasoning (TasteNrich^{®}, TNR, CJ CheilJedang) as a nitrogen source at 1.2% by weight, and these were mixed together. Next, the *Leuconostoc mesenteroides* CJLM119 strain (accession number KCTC 13043BP, US 2019-0297908 A1) or *Leuconostoc mesenteroides* KCTC 3505 was added at 2% by weight, water was added until the total weight became 100% by weight, mixing was performed, and then culturing was performed at 30°C for 20 hours for fermentation, whereby fermented compositions of Preparation Examples 1 to 4 and Comparative Examples 1 to 3 were prepared. After fermentation, the bacterial count of *Leuconostoc mesenteroides* CJLM119 strain in each fermented composition was 2 × 10⁸ cfu/mL.

The supernatant of the fermented composition after completion of fermentation was obtained by centrifuging the fermented composition at 13,000 rpm for 5 minutes and filtering the supernatant through a 0.45 µm syringe filter to remove the bacteria. The *Leuconostoc mesenteroides* strain in the fermented composition was consistently 10⁸ cfu/mL or more.

**[Table 1]**

| Raw material | Added amount (wt%) | Preparati on Example 1 (garlic after fermentat ion) | Preparati on Example 2 (red pepper powder) | Preparati on Example 3 (TNR) | Compara tive Example 1 (all materials ) | Compara tive Example 2 (garlic before fermentat ion) | Preparati on Example 4 (fermente d compositi on of garlic+ red pepper powder + seasonin g with LM119) | Compara tive Example 3 (fermente d compositi on of garlic+ red pepper powder + seasonin g with KCTC 3505) |
|---|---|---|---|---|---|---|---|---|
| LM119 strain | 2 | O | O | O | **○** | - | **○** | - |
| KCTC 3505 strain | 2 | - | - | - | - | - | - | O |
| Crystallin e fructose | 3 | O | O | O | O | O | O | O |
| Salt | 1.5 | O | O | O | O | O | O | O |
| Garlic | 7 | O | - | - | - | O | O | O |
| Red pepper powder | 2 | - | O | - | - | O | O | **○** |
| Season in g (TNR) | 1.2 | - | - | O | - | O | O | O |
| Ginger | 1.2 | - | - | - | - | - | O | O |
| Water | Remaind er | O | O | O | O | O | O | O |
| Total (wt%) | 100 | 100 | 100 | 100 | 100 | 100 | 100 | 100 |

### Example 1: Examination of cytotoxicity of fermented composition

Cytotoxicity of each fermented composition was examined using the WST-1 assay (Roche). The WST-1 assay was performed using the cell proliferation reagent WST-1 assay kit (Roche, USA) according to the manufacturer's instructions.

Specifically, J774A.1, a mouse macrophage cell line, obtained from the Korean Cell Line Bank (KCLB) was used as an animal cell. The cells were cultured at 37°C in the presence of 5% CO₂ using DMEM (Gibco, USA) supplemented with heat-inactivated 10% fetal bovine serum (FBS), 1% L-glutamine, penicillin G (100 IU/mL), and streptomycin (100 mg/mL). The cells were dispensed into 96-well plates (Corning, USA) at 5 × 10⁴ cells/well (volume 150 µL) per well, cultured for about 3 hours until the cells completely formed a monolayer, and used in the experiment.

### 1-1. Cytotoxicity of fermented composition by material

The fermented compositions of Preparation Examples 1 to 3 and Comparative Examples 1 and 2 were diluted with DMEM medium supplemented with no antibiotics, and dispensed into 96-well plates by 100 µL. As controls, DMEM medium supplemented with no antibiotics (negative control) or DMEM medium supplemented with 1 µg/mL of lipopolysaccharide (LPS) and no antibiotics (positive control) was dispensed into 96-well plates by 100 µL. J774A. 1 cells were seeded so as to be 3.3 × 10⁵ cells/mL in each fermented composition or control. The 96-well plates were incubated at 37°C for 24 hours in the presence of 5% CO₂.

After completion of culturing, the cell supernatant was removed, WST-1 was diluted with DMEM to be 10%, and dispensed into each well by 100 µL, and culturing was performed at 37°C for 1 hour in the presence of 5% CO₂. Afterwards, the absorbance was measured at 500 nm using a spectrophotometer (BioTek, USA).

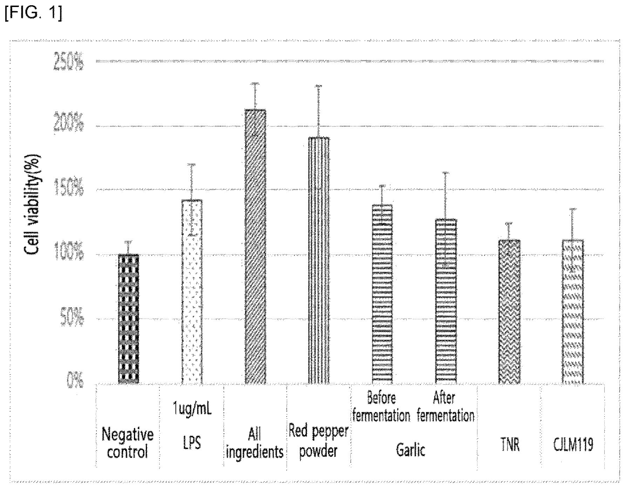

As a result, in all cells treated with the fermented compositions of Preparation Examples 1 to 3 and Comparative Examples 1 and 2, the cell viability increased compared to that in the negative control, and it has been found that the fermented compositions do not exhibit cytotoxicity (FIG. 1).

### 1-2. Cytotoxicity of fermented composition

The fermented composition of Preparation Example 4 was diluted with DMEM medium supplemented with no antibiotics so that the *Leuconostoc mesenteroides* CJLM119 strain in the fermented composition was 5 × 10⁶ cfu/mL (fermented composition A), 10⁶ cfu/mL (fermented composition B), and 5 × 10⁵ cfu/mL (fermented composition C), and these were dispensed into 96-well plates by 100 µL. The supernatants of the fermented compositions A to C were diluted with DMEM supplemented with no antibiotics at the same dilution factor, and these were dispensed into 96-well plates by 100 µL. As controls, DMEM medium supplemented with no antibiotics (negative control) or DMEM medium supplemented with 1 µg/mL of LPS and no antibiotics (positive control) were dispensed into 96-well plates by 100 µL. J774A.1 cells were seeded so as to be 3.3 × 10⁵ cells/mL in each fermented composition, its supernatant or each control. The 96-well plates were incubated at 37°C for 24 hours in the presence of 5% CO₂. After culturing, the absorbance was measured at 500 nm using a spectrophotometer in the same manner as in Example 1-1.

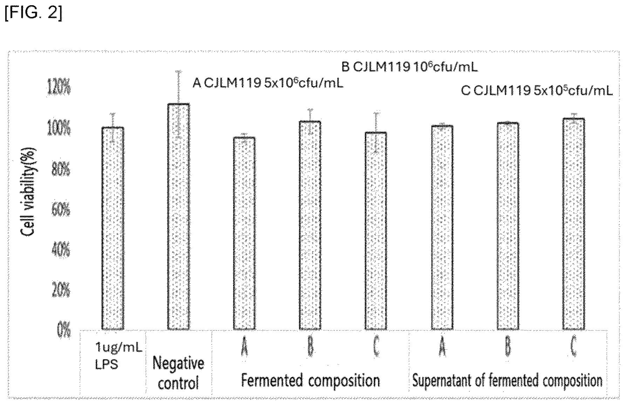

As a result, the cell viability was maintained in cells treated with the fermented compositions containing the *Leuconostoc mesenteroides* CJLM119 strain at 5 × 10⁶ cfu/mL (fermented composition A), 10⁶ cfu/mL (fermented composition B), and 5 × 10⁵ cfu/mL (fermented composition C) and the supernatants thereof, and it has been found that the fermented compositions and the supernatants thereof do not exhibit cytotoxicity (FIG. 2).

### 1-3. Cytotoxicity of fermented composition by fermentation strain

The fermented composition of Preparation Example 4 was diluted with DMEM medium supplemented with no antibiotics so that the *Leuconostoc mesenteroides* CJLM119 strain in the fermented composition was 1.25 × 10⁶ cfu/mL, 2.5 × 10⁶ cfu/mL, 5.0 × 10⁶ cfu/mL, and 6.25 × 10⁶ cfu/mL, and these were dispensed into 96-well plates by 100 µL. Comparative Example 3 was diluted with DMEM medium supplemented with no antibiotics so that the *Leuconostoc mesenteroides* KCTC 3505 strain in the fermented composition was 1.25 × 10⁶ cfu/mL, 2.5 × 10⁶ cfu/mL, 5.0 × 10⁶ cfu/mL, and 6.25 × 10⁶ cfu/mL, and these were dispensed into 96-well plates by 100 µL. As controls, DMEM medium supplemented with no antibiotics (negative control) or DMEM medium supplemented with 1 µg/mL of LPS and no antibiotics (positive control) were dispensed into 96-well plates by 100 µL. J774A.1 cells were seeded so as to be 3.3 × 10⁵ cells/mL in each fermented composition or control. The 96-well plates were incubated at 37°C for 24 hours in the presence of 5% CO₂. After culturing, the absorbance was measured at 500 nm using a spectrophotometer in the same manner as in Example 1-1.

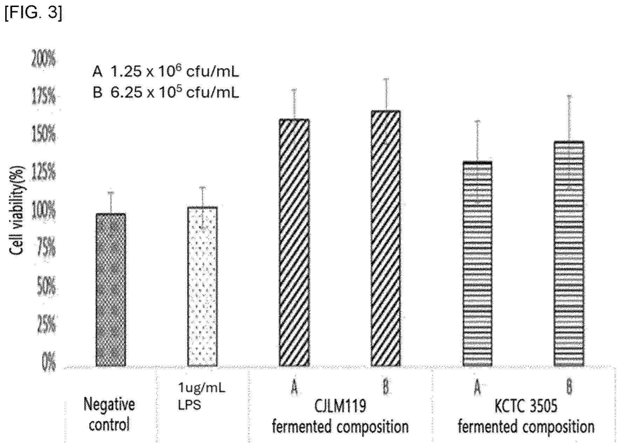

As a result, in all cells treated with the fermented compositions containing the *Leuconostoc mesenteroides* CJLM119 or KCTC 3505 strain at 1.25 × 10⁶ cfu/mL and 6.25 × 10⁶ cfu/mL, the cell viability increased compared to that in the negative control, and it has been found that the fermented compositions do not exhibit cytotoxicity (FIG. 3).

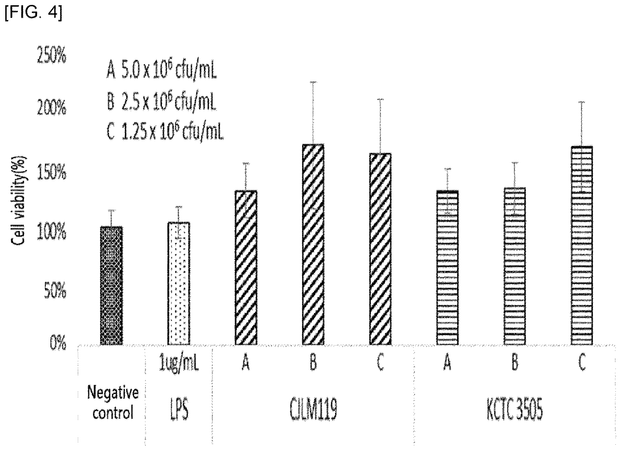

In addition, in all cells treated with the fermented compositions containing the *Leuconostoc mesenteroides* CJLM119 or KCTC 3505 strain at 1.25 × 10⁶ cfu/mL, 2.5 × 10⁶ cfu/mL, and 5.0 × 10⁶ cfu/mL, the cell viability increased compared to that in the negative control, and it has been found that the fermented compositions do not exhibit cytotoxicity (FIG. 4).

### Example 2: Examination of immune enhancing effect of fermented composition

In order to evaluate the immune enhancing effect of the fermented compositions of Preparation Examples 1 to 4, the ability of the fermented compositions to secrete tumor necrosis factor-α (TNF-α) and interleukin-6 (IL-6), Th1 type pro-inflammatory cytokines, was examined by the ELISA assay.

### 2-1. Immune enhancing effect of fermented composition by material

The fermented compositions of Preparation Examples 1 to 3 and Comparative Examples 1 and 2 were diluted with DMEM medium supplemented with no antibiotics, and dispensed into 96-well plates by 100 µL. As controls, DMEM medium supplemented with no antibiotics (negative control) or DMEM medium supplemented with 1 µg/mL of LPS and no antibiotics (positive control) were dispensed into 96-well plates by 100 µL. J774A.1 cells were seeded so as to be 3.3 × 10⁵ cells/mL in each fermented composition or control. The 96-well plates were incubated at 37°C for 24 hours in the presence of 5% CO₂.

After completion of culturing, the culture solution was centrifuged at 1,400 rpm for 10 minutes to obtain a cell supernatant from which J774A.1 cells and *Leuconostoc mesenteroides* strain were removed. The ELISA assay was performed using the DuoSet ELISA ancillary reagent kit 2 (R&D systems, cat DY008, USA). The Mouse TNF-α DuoSet ELISA kit (R&D systems, cat number DY 406-05, USA) containing TNF-α antibody and biotinylated TNF-α antibody, a secondary antibody, and Mouse IL-6 DuoSet ELISA kit (R&D systems, cat number DY 410-05, USA) containing IL-6 antibody and biotinylated IL-6 antibody, a secondary antibody, were used according to the manufacturer's instructions. The ELISA plate was coated with TNF-α capture antibody (R&D systems, cat number 840143) and IL-6 capture antibody (R&D systems, cat number 840171) at room temperature for 24 hours, respectively. Thereafter, 100 µL of cell supernatant and TNF-α standard solution (R&D systems, cat number 840145) or IL-6 standard solution (R&D systems, cat number 840173) were diluted with a reaction diluent (R&D systems, cat number DY995), added to the plate by 100 µL, and cultured at room temperature for 2 hours. Afterwards, the culture solution was removed, and biotinylated TNF-α antibody (R&D systems, cat number 840144) and biotinylated IL-6 antibody (R&D systems, cat number 840172), secondary antibodies, were added to coat the plate at room temperature for 2 hours. The color reaction was induced by streptavidin-conjugated horseradish peroxidase (R&D systems, cat number 893975) and TMB (tetramethylbenzidine, R&D systems, cat number DY999), its substrate. After the color reaction proceeded, 50 µL of stop solution (2 N H₂SO₄) was added to each well to stop the reaction. At this time, the color reaction changed from blue to yellow, and after completion of the color reaction, the absorbance was measured at 470 nm (correction value 540 nm) using a spectrophotometer.

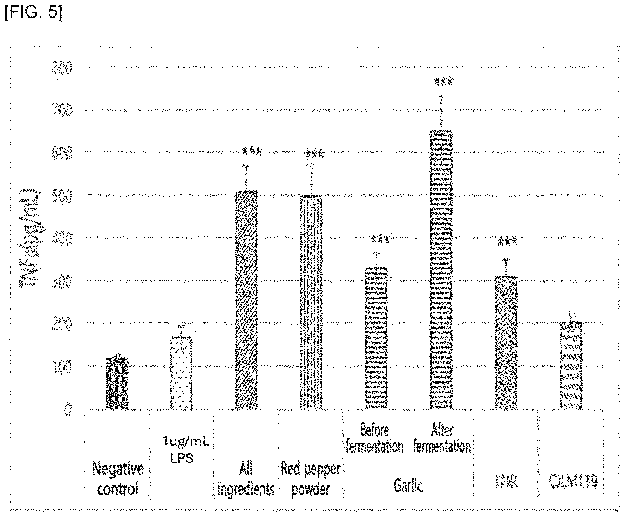

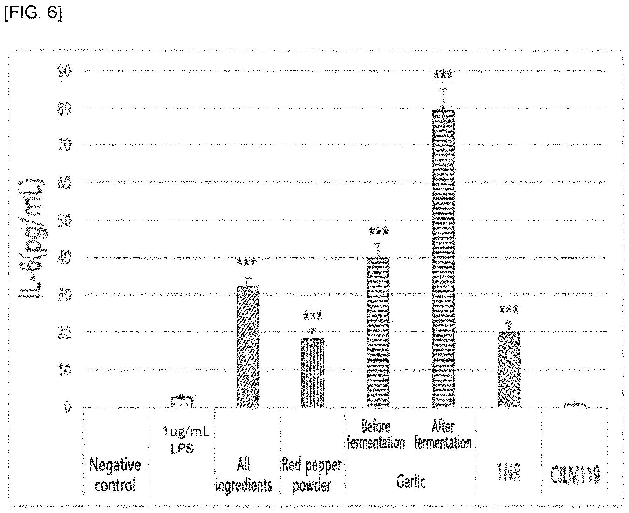

As a result, in cells treated with the fermented compositions of Preparation Examples 1 to 3 and Comparative Examples 1 and 2, the expression of TNF-α (FIG. 5) and IL-6 (FIG. 6) was significantly increased compared to that in the negative control group (***p < 0.001).

In particular, in the case of the fermented composition of Preparation Example 1 obtained by fermenting a composition containing garlic with the *Leuconostoc mesenteroides* CJLM119 strain, the expression of TNF-α and IL-6 was significantly increased compared to that in the case of the other fermented compositions, and it has been found that the fermented composition of Preparation Example 1 exhibits an excellent immune enhancing effect, and the expression of TNF-α and IL-6 was significantly improved compared to that in the case of Comparative Example 2 in which a composition containing garlic was not fermented with the *Leuconostoc mesenteroides* CJLM119 strain.

Specifically, as illustrated in FIG. 5, the expression of IL-6 in cells treated with Comparative Example 2 in which a composition containing garlic was not fermented with the *Leuconostoc mesenteroides* CJLM119 strain was higher compared to that in cells treated with the other fermented compositions and the controls, but the expression of IL-6 increased about two-fold in cells treated with Preparation Example 1 in which a composition containing garlic was fermented with the *Leuconostoc mesenteroides* CJLM119 strain, and it has been found that the composition fermented with the *Leuconostoc mesenteroides* CJLM119 strain exhibits a significantly excellent immune enhancing effect.

### 2-2. Immune enhancing effect of fermented composition

The fermented composition of Preparation Example 4 was diluted with DMEM medium supplemented with no antibiotics so that the *Leuconostoc mesenteroides* CJLM119 strain in the fermented composition was 5 × 10⁶ cfu/mL, 10⁶ cfu/mL, and 5 × 10⁵ cfu/mL, and these were dispensed into 96-well plates by 100 µL. J774A.1 cells were seeded so as to be 3.3 × 10⁵ cells/mL in each fermented composition. The 96-well plates were incubated at 37°C for 24 hours in the presence of 5% CO₂. After culturing, the color reaction was completed in the same manner as in Example 2-1, and then the absorbance was measured at 470 nm (correction value 540 nm) using a spectrophotometer.

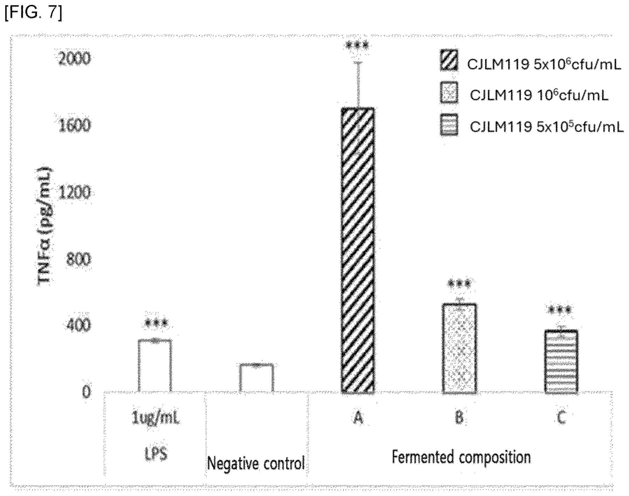

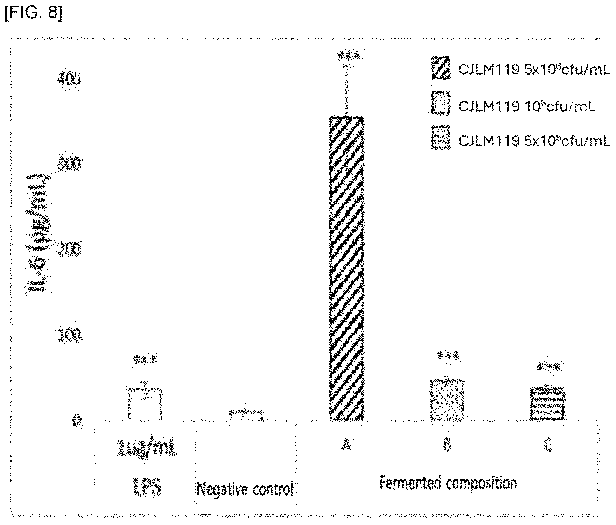

As a result, in cells treated with the fermented compositions containing the *Leuconostoc mesenteroides* CJLM119 strain at 5 × 10⁶ cfu/mL, 10⁶ cfu/mL, and 5 × 10⁵ cfu/mL, the expression of TNF-α (FIG. 7) and IL-6 (FIG. 8) was significantly increased compared to that in the negative control or similar to that in the negative control (***p < 0.001, **p < 0.01).

In particular, in the case of the fermented composition A containing the *Leuconostoc mesenteroides* CJLM119 strain at 5 × 10⁶ cfu/mL, the expression of TNF-α and IL-6 was significantly improved compared to that in the case of the other fermented compositions, and it has been found that the fermented composition A exhibits an excellent immune enhancing effect.

### 2-3. Immune enhancing effect of fermented composition by fermentation strain

The fermented composition of Preparation Example 4 was diluted with DMEM medium supplemented with no antibiotics so that the *Leuconostoc mesenteroides* CJLM119 strain in the fermented composition was 1.25 × 10⁶ cfu/mL, 2.5 × 10⁶ cfu/mL, 5.0 × 10⁶ cfu/mL, and 6.25 × 10⁶ cfu/mL, and these were dispensed into 96-well plates by 100 µL. Comparative Example 3 was diluted with DMEM medium supplemented with no antibiotics so that the *Leuconostoc mesenteroides* KCTC 3505 strain in the fermented composition was 1.25 × 10⁶ cfu/mL, 2.5 × 10⁶ cfu/mL, 5.0 × 10⁶ cfu/mL, and 6.25 × 10⁶ cfu/mL, and these were dispensed into 96-well plates by 100 µL. As controls, DMEM medium supplemented with no antibiotics (negative control) or DMEM medium supplemented with 1 µg/mL of LPS and no antibiotics (positive control) were dispensed into 96-well plates by 100 µL. J774A.1 cells were seeded so as to be 3.3 × 10⁵ cells/mL in each fermented composition or control. The 96-well plates were incubated at 37°C for 24 hours in the presence of 5% CO₂. After culturing, the color reaction was completed in the same manner as in Example 2-1, and then the absorbance was measured at 470 nm (correction value 540 nm) using a spectrophotometer.

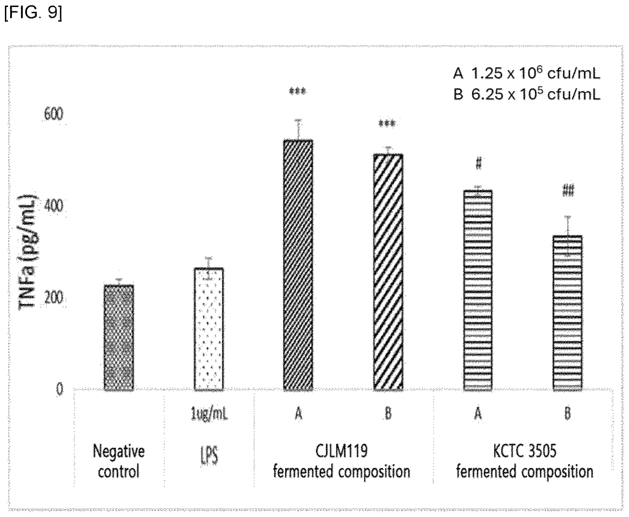

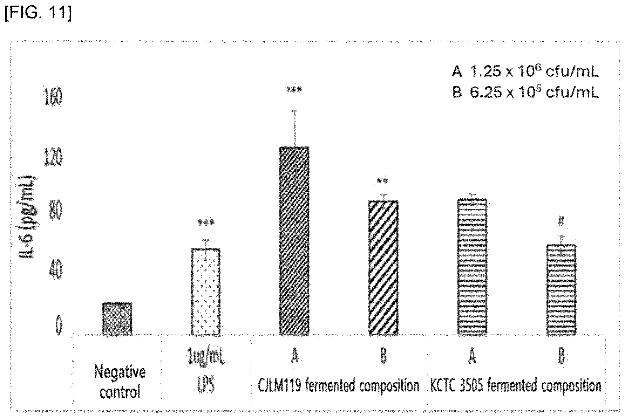

As a result, in all cells treated with the fermented compositions containing the *Leuconostoc mesenteroides* CJLM119 strain at 1.25 × 10⁶ cfu/mL and 6.25 × 10⁶ cfu/mL, the expression of TNF-α (FIG. 9) and IL-6 (FIG. 11) was significantly increased compared to that in the negative control (***p < 0.001). In cells treated with the fermented compositions containing the *Leuconostoc mesenteroides* KCTC 3505 strain at 1.25 × 10⁶ cfu/mL and 6.25 × 10⁶ cfu/mL, the expression of TNF-α was significantly lower compared to that in cells treated with the LM119 fermented compositions (1.25 × 10⁶ cfu/mL and 6.25 × 10⁶ cfu/mL) and the expression of IL-6 was significantly lower compared to that in cells treated with the LM119 fermented composition (1.25 × 10⁶ cfu/mL) (FIGS. 9 and 11, ##p < 0.01, #p < 0.05).

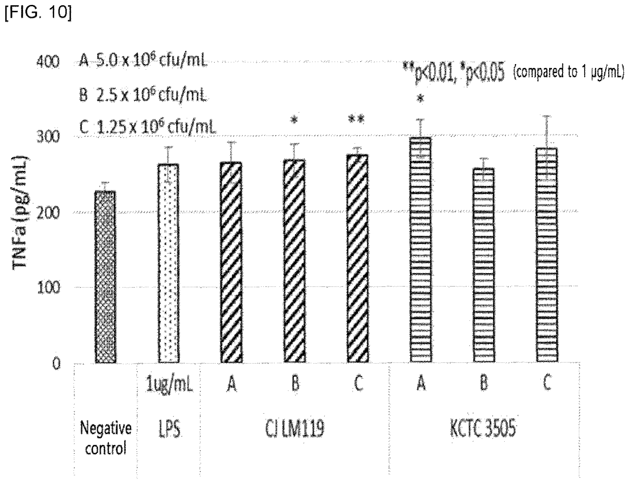

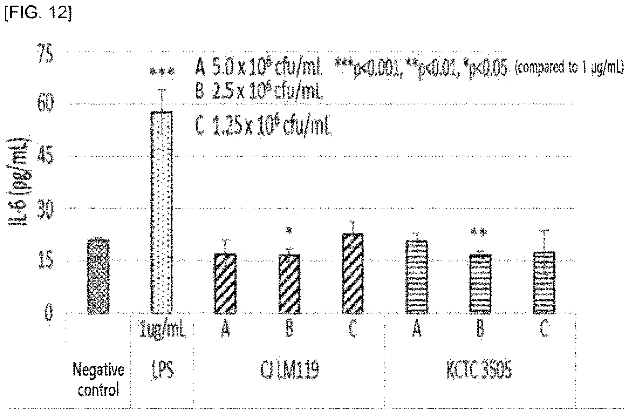

In addition, in all cells treated with the fermented compositions containing the *Leuconostoc mesenteroides* CJLM119 or KCTC 3505 strain at 1.25 × 10⁶ cfu/mL, 2.5 × 10⁶ cfu/mL, and 5.0 × 10⁶ cfu/mL, the expression of TNF-α (FIG. 10) and IL-6 (FIG. 12) was significantly increased compared to that in the negative control (***p < 0.001, **p < 0.01, *p < 0.05). In cells treated with the fermented compositions containing the *Leuconostoc mesenteroides* KCTC 3505 strain at 2.5 × 10⁶ cfu/mL, the expression of TNF-α was significantly lower compared to that in cells treated with the LM119 fermented composition (2.5 × 10⁶ cfu/mL) and the expression of IL-6 was significantly lower compared to that in cells treated with the LM119 fermented compositions (2.5 × 10⁶ cfu/mL and 5.0 × 10⁶ cfu/mL) (FIGS. 10 and 12, ***p < 0.001, **p < 0.01, *p < 0.05).

### Example 3: Examination of effect of inhibiting growth of harmful bacteria of fermented composition by material

In order to examine the effect of inhibiting the growth of harmful bacteria in the fermented composition of Preparation Example 4, *Escherichia coli* and *Staphylococcus aureus,* pathogenic microorganisms known as bacteria that cause food poisoning, were each inoculated into BHI (brain heart infusion) broth medium (BD DIFCO) and cultured at 37°C for 24 hours to obtain culture. The fermented composition of Preparation Example 4 was diluted with BHI broth medium so as to have concentrations of 0%, 25%, 40%, and 50% (v/v), thereby preparing the media. The diluted media were each dispensed into 96-well plates by 200 µl, and *E*. *coli* and *S*. *aureus* strains were each seeded at 1 × 10⁶ cells/well. The 96-well plates were incubated at 37°C for 24 hours. After culturing, the OD₆₀₀ value was measured using a spectrophotometer. By comparing the OD₆₀₀ value in a medium containing the fermented composition at 0% and the OD₆₀₀ value in the diluted medium containing the fermented composition at 25%, 40%, or 50% (v/v), the growth inhibition ratio (%) of harmful bacteria at each concentration of the fermented composition was examined. The growth inhibition ratio was calculated from the following equation, and the OD₆₀₀ value of each diluted medium inoculated with no strain was taken as the blank. Inhibition ratio (%) = [1 - (OD600 of experimental group inoculated with strain and after culturing) - (OD600 of experimental group inoculated with no strain and after culturing) / (OD600 of control inoculated with strain and after culturing) - (OD600 of control inoculated with no strain and after culturing)] × 100(%)

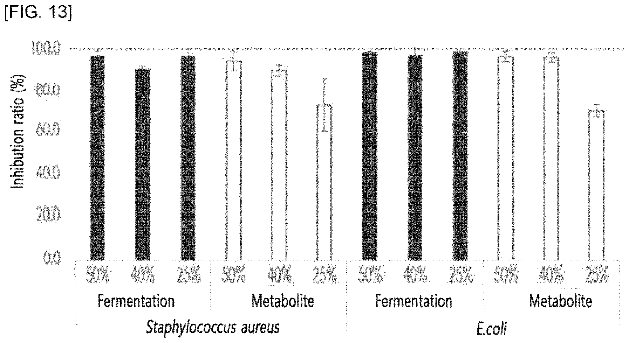

As a result, the fermented composition of Preparation Example 1 exhibited a growth inhibition ratio as high as 90% or more at all concentrations against *E*. *coli* and *S*. *aureus,* bacteria that cause food poisoning (FIG. 13).

Based on the above description, it will be understood by those skilled in the technical field to which the present disclosure belongs that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within the metes and bounds of the claims or equivalents of such metes and bounds are therefore intended to be embraced by the claims.

## Claims

1. A composition for fermentation, comprising any one or more selected from a *Leuconostoc mesenteroides* CJLM119 strain or a culture thereof.

2. A food composition for immune enhancement, comprising a fermentation product fermented with a *Leuconostoc mesenteroides* CJLM119 strain or a culture thereof.

3. A food composition for gut health improvement, comprising a fermentation product fermented with a *Leuconostoc mesenteroides* CJLM119 strain or a culture thereof.

4. The food composition according to claim 2 or 3, wherein the fermentation product is a product in which a composition comprising any one or more selected from the group consisting of garlic, red pepper powder, and a nitrogen source is fermented.

5. The food composition according to claim 2 or 3, wherein the fermentation product is a product in which a composition comprising garlic at 5% to 9% by weight with respect to a total weight of the composition is fermented.

6. The food composition according to claim 2 or 3, wherein the food composition increases expression of TNF-α or IL-6.

7. The food composition according to claim 2 or 3, wherein the food composition inhibits growth of harmful bacteria.

8. The food composition according to claim 7, wherein the harmful bacteria are any one or more selected from the group consisting of *Escherichia sp., Staphylococcus sp., Salmonella sp., Shigella sp., Klebsiella sp., Enterobacteriaceae sp., Pseudomonas sp., Moraxella sp., Helicobacter sp., Stenotrophomonas sp., Listeria sp.,* and *Vibrio sp..*

9. The food composition according to claim 2 or 3, wherein the fermentation product is a product fermented at 32°C to 42°C for 10 to 30 hours.

10. The food composition according to claim 2 or 3, wherein the fermentation product is a product in which a composition further comprising any one or more selected from a carbon source or salt is fermented.

11. The food composition according to claim 2 or 3, wherein the fermentation product comprises any one or more selected from a culture of *Leuconostoc mesenteroides* CJLM119 strain, a lysate thereof, an extract thereof, or a component derived therefrom.

12. The food composition according to claim 2 or 3, wherein the fermentation product comprises a fermentation product fermented with a *Leuconostoc mesenteroides* CJLM1 19 strain or a culture thereof; or a fermentation product in which the fermentation product is further fermented.

13. A health functional food for immune enhancement, comprising a fermentation product fermented with a *Leuconostoc mesenteroides* CJLM119 strain or a culture thereof.

14. A health functional food for gut health improvement, comprising a fermentation product fermented with a *Leuconostoc mesenteroides* CJLM119 strain or a culture thereof.

15. The health functional food according to claim 12 or 13, wherein the fermentation product is a product in which a composition comprising any one or more selected from the group consisting of garlic, red pepper powder, and a nitrogen source is fermented.

16. The health functional food according to claim 12 or 13, wherein the fermentation product is a product in which a composition comprising garlic at 5% to 9% by weight with respect to a total weight of the composition is fermented.

17. The health functional food according to claim 12 or 13, wherein the fermentation product comprises a fermentation product fermented with a *Leuconostoc mesenteroides* CJLM119 strain or a culture thereof; or a fermentation product in which the fermentation product is further fermented.

18. A method for increasing an immune enhancing effect of a composition, the method comprising fermenting the composition with a *Leuconostoc mesenteroides* CJLM119 strain or a culture thereof.

19. A method for increasing a gut health improving effect of a composition, the method comprising fermenting the composition with a *Leuconostoc mesenteroides* CJLM119 strain or a culture thereof.

20. The method according to claim 18 or 19, wherein the composition further comprises any one or more selected from the group consisting of garlic, red pepper powder, and a nitrogen source.

21. The method according to claim 18 or 19, wherein the composition is a food composition.
